# EUROPEAN PATENT SPECIFICATION

(11) **EP 2 015 809 B1**
(45) Date of publication and mention of the grant of the patent: **04.11.2015**
(21) Application number: 07732740.1
(22) Date of filing: 10.05.2007
(51) Int. Cl.: A61M 5/32, A61M 5/50

(54) **NEEDLE TIP STORAGE AND REMOVAL DEVICE**
NADELSPITZEN-AUFBEWAHRUNGS- UND ENTFERNUNGSVORRICHTUNG
DISPOSITIF DE STOCKAGE ET DE RETRAIT D'UNE POINTE D'AIGUILLE

(30) Priority: 11.05.2006 GB 0609308
(43) Date of publication of application: 21.01.2009
(60) Divisional application: 15186334.7
(73) Proprietor: OWEN MUMFORD LIMITED, Woodstock, Oxford OX20 ITU (GB)
(72) Inventor: CROSSMAN, David, Danvers, Oxford OX9 5HL (GB); ROLFE, Steven, Mark, Guy, Oxon OX27 8DF (GB)
(74) Representative: Wynne-Jones, Laine and James LLP
(86) International application number: PCT/GB2007/001712
(87) International publication number: WO 2007/132185

(56) References cited:
- EP-A- 1 741 459
- WO-A-2005/011781
- US-A- 5 026 345
- US-A- 5 554 129
- US-B1- 6 315 113

## Description

This invention relates to a needle tip storage and removal device and in particular, but not exclusively, to a device for use with an injection device intended to inject a number of metered doses from a cartridge of therapeutic material.

In a conventional pen injector such as the Owen Mumford Autopen^{®}, a cartridge of therapeutic substance is loaded into the cartridge holder and a single use needle tip is screwed onto the forward end of the device. The needle tip has a double-ended needle, the rear end of which penetrates a rubber membrane in the forward end of the cartridge as the needle tip is screwed on. A metered dose can then be dialled into the pen injector, the needle pushed into the injection site, and the trigger on the device actuated to inject the metered dose. After the injection, the needle tip should be removed from the device and discarded.

In many conventional arrangements, the exterior of the needle tip hub is provided with splines and the needle tip is supplied in a foil-sealed container, having a complementary splined socket in which the needle hub is supplied. To use such a needle tip, the foil is removed and the needle, whilst still shrouded by the container is screwed onto the injection device using the container as a spanner. Once fully home, the container may be withdrawn axially leaving the needle exposed, although in some instances a removable secondary needle shield is provided. Following injection, the user may slide the container back onto the needle hub and use it as a spanner to remove the needle tip. The needle tip can then be safely discarded because the used needle is now re-enclosed by the container. This system works extremely well provided the user retains the container and uses it to remove the needle tip immediately after the injection. However, in many instances, users may not follow the recommendation to change the needle tip after each injection and may instead use the same needle tip for several injections over an extended period. In this case they may have discarded the original container and so may be left to remove the needle tip by unscrewing it whilst the needle is still exposed. This provides a risk of needle stick injury both whilst removing the needle tip and when it is discarded. Alternatively, they may inject themselves and then leave the needle in place, covering it with the device cap, and then remove the needle only when they wish to replace with a new needle. Again, this poses a risk of needle stick injury.

We have designed an arrangement to reduce these risks.

US5,554,129 discloses a free-standing safety cap arrangement having a first compartment for temporarily securing an unused medical instrument and a second compartment for permanently securing a used medical instrument. The first and second compartments each make different connections with the medical instrument.

US5,026,345 discloses a needle guard filled with a liquid adhesive to lock the needle guard permanently to the syringe after use.

WO2005/011781 discloses an arrangement having first and second cylindrical chambers in one of which is disposed a safety needle assembly and in the other of which is permanently secured a vial loader.

EP1741459 published after the priority date of this application discloses an arrangement having a first housing accommodating at least one needle and a second housing.

US6315113 discloses a disposing cap for enclosing and protecting a needle and having a separate chamber containing a substance to dissolve the needle after use.

Accordingly, this invention provides a needle tip storage and removal device comprising a storage compartment for receiving in use a needle tip of a given drive configuration comprising a threaded collar with an externally splined cylindrical portion, said storage compartment having a complementarily splined drive socket for non-rotatably engaging the splined portion of a needle tip received in use in said storage compartment, said device further including a further drive socket adapted to be fitted onto a needle tip of said given drive configuration, wherein said further drive socket is complementarily splined for receiving in use the externally splined portion of a used needle tip of said given configuration, said further drive socket being provided in a removal compartment arranged alongside the storage compartment, and said compartments are arranged side by side and advantageously with their open ends facing in opposite directions.

In this manner, if a user finds that they have lost the original container when they wish to replace a needle tip, they can use the drive socket on the new container.

The storage and removal compartments are preferably open at one end to receive the needle tip and closed at the other end to shroud the exposed needle.

Preferably said device comprises a single storage compartment and a single removal compartment.

The invention also extends to a needle tip storage and removal device as described above, in combination with a needle tip located in said storage compartment, the storage compartment being sealed by a removable sealing element, and the removal compartment being initially empty.

The invention may be performed in various ways, and an embodiment thereof will now be described by way of example only, reference being made to the accompanying drawings, in which:

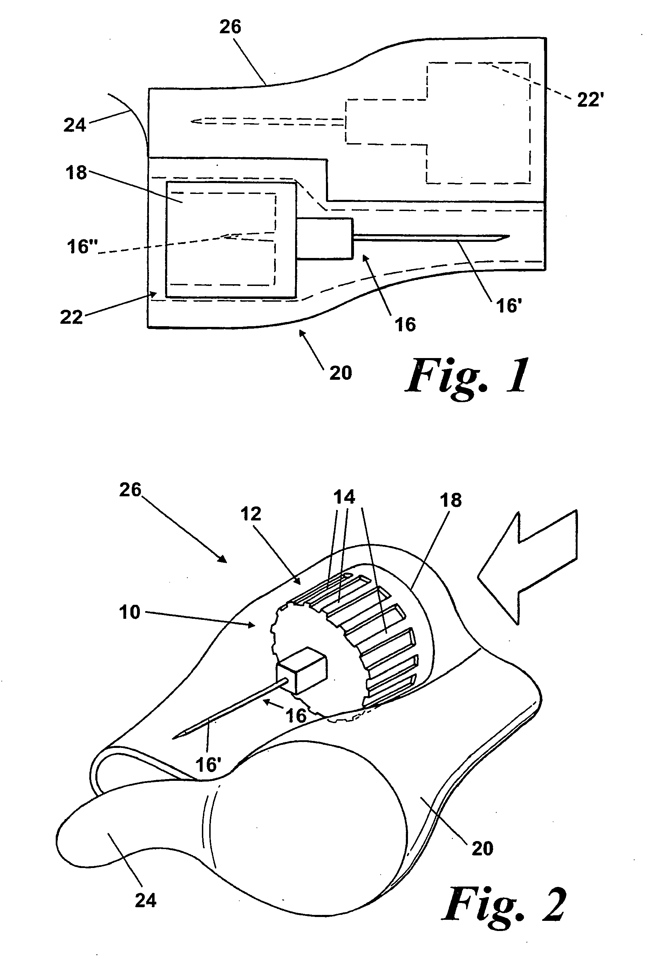

Figure 1 is a schematic top plan view of a needle tip storage and removal device in accordance with this invention, and
Figure 2 is a general perspective view of the device of Figure 1 with a used needle tip in the removal compartment.

The device illustrated in the Figures is intended to store a needle tip 10 for being screwed onto the end of a pen injector type device, and to remove the needle tip after use and to shield the forward part of the needle after use.

The needle tip may be a Unifine^{®} pen tip. The pen tip comprises a threaded collar 12 having external splines 14 with a double ended needle 16 having a forward part 16' projecting forwardly of the collar 18 and a rearward part 16" extending rearwardly but not projecting beyond the rearward surface of the collar. The device in this embodiment includes a storage compartment 20 having a complementarily splined socket 22 into which the drive collar can be slid with the needle guarded by the distal end of the compartment 20. The storage compartment 20 is sealed by a peelable sealing foil element 24. Alongside the storage compartment 20 is a removal compartment 26 which also has a complementarily splined socket 22' and a needle shrouding portion and is generally similar to the configuration of the storage compartment, although the removal compartment may be designed to have snap lock means or the like to permanently capture the used needle tip.

In use, assuming that the pen injector already has a used needle attached, the removal compartment 26 is slid over the used needle and turned to unscrew the needle tip and to leave it in the removal compartment, with the used exposed needle shrouded by the compartment. Having done this, the foil 24 may be peeled off the storage compartment 20 and the new needle tip 10 screwed onto the device. The needle tip storage and removal device can then be discarded.

## Claims

1. A needle tip storage and removal device comprising a storage compartment for receiving in use a needle tip (10) of a given drive configuration comprising a threaded collar (12) with an externally splined cylindrical portion (14), said storage compartment (20) having a complementarily splined drive socket for non-rotatably engaging the splined portion of a needle tip received in use in said storage compartment, said device further including a further drive socket (22¹) adapted to be fitted onto a needle tip (10) of said given drive configuration, **characterised in that** said further drive socket (22¹) is complementarily splined for receiving in use the externally splined portion of a used needle tip of said given configuration, said further drive socket being provided in a removal compartment arranged alongside the storage compartment (20), and wherein said compartments are arranged with their open ends facing in opposite directions.

2. A needle tip storage and removal device according to Claim 2, wherein said compartments are open at one end to receive the needle tip and closed at the other end.

3. A needle tip storage and removal device according to any of the preceding Claims, with a needle tip (10) located in said storage compartment, the storage compartment being sealed by a removable sealing foil element.

## Patentansprüche

1. Nadelspitzen-Aufbewahrungs- und Entfernungs-Vorrichtung, umfassend eine Kammer bei Benutzung zur Aufnahme einer Nadelspitze (10) mit einer gegebenen Antriebskonfiguration, die einen genuteten Kragen (12) aufweist, der einen mit einem außenkerbverzahnten, zylindrischen Teil (14) versehen ist, wobei die Aufbewahrungskammer (20) eine komplementär kerbverzahnte Antriebshülse besitzt, die mit dem kerbverzahnten Teil einer Nadelspitze, welche bei Benutzung in der Aufbewahrungskammer sitzt, in nicht drehbarem Eingriff steht, und wobei die Vorrichtung des weiteren eine weitere Antriebshülse (22') aufweist, die auf eine Nadelspitze (10) der gegebenen Antriebskonfiguration gesetzt werden kann, **dadurch gekennzeichnet, daß** die weitere Antriebshülse (22') komplementär kerbverzahnt ist und bei Benutzung zwecks Aufnahme des äußerlich kerbverzahnten Teils einer benutzten Nadelspitze der gegebenen Konfiguration dient, daß des weiteren die Antriebshülse in einer entfernbaren Kammer längs der Speicherkammer (20) angeordnet ist, und daß die Kammern mit ihren offenen Enden so angeordnet sind, daß sie in entgegengesetzte Richtungen weisen.

2. Nadelspitzen-Aufbewahrungs- und Entfernungs-Vorrichtung nach Anspruch 1, **dadurch gekennzeichnet, daß** die Kammern an dem einen Ende zur Aufnahme der Nadelspitze offen und an dem anderen Ende geschlossen sind.

3. Nadelspitzen-Aufbewahrungs- und Entfernungs-Vorrichtung nach einem der vorhergehenden Ansprüche, **dadurch gekennzeichnet, daß** dann, wenn eine Nadelspitze (10) in der Aufbewahrungskammer liegt, die Aufbewahrungskammer durch ein entfernbares Dichtungsfolienelement verschlossen wird.

## Revendications

1. Dispositif de stockage et de retrait de pointe d'aiguille comprenant un compartiment de stockage pour recevoir en utilisation une pointe d'aiguille (10) d'une configuration d'entraînement donnée comprenant un collet fileté (12) avec une partie cylindrique à cannelures externes (14), ledit compartiment de stockage (20) ayant un embout femelle d'entraînement à cannelures complémentaires pour engager de façon non-rotative la partie cannelée d'une pointe d'aiguille reçue en utilisation dans ledit compartiment de stockage, ledit dispositif comprenant en outre un autre embout femelle d'entraînement (22¹) apte à être adapté sur une pointe d'aiguille (10) de ladite configuration d'entraînement donnée, **caractérisé par le fait que** ledit autre embout femelle d'entraînement (22¹) est cannelé de façon complémentaire pour recevoir en utilisation la partie à cannelures externes d'une pointe d'aiguille usagée de ladite configuration donnée, ledit autre embout femelle d'entraînement étant disposé dans un compartiment de retrait agencé le long du compartiment de stockage (20), et lesdits compartiments étant agencés avec leurs extrémités ouvertes tournées dans des directions opposées.

2. Dispositif de stockage et de retrait de pointe d'aiguille selon la revendication 1, dans lequel lesdits compartiments sont ouverts à une extrémité pour recevoir la pointe d'aiguille et fermés à l'autre extrémité.

3. Dispositif de stockage et de retrait de pointe d'aiguille selon l'une quelconque des revendications précédentes, avec une pointe d'aiguille (10) située dans ledit compartiment de stockage, le compartiment de stockage étant scellé de manière étanche par un élément de feuille de scellement amovible.
